(19) Europäisches Patentamt
European Patent Office
Office européen des brevets

(11) EP 1 652 510 B1

(12) **FASCICULE DE BREVET EUROPEEN**

(45) Date de publication et mention de la délivrance du brevet:
**09.04.2008 Bulletin 2008/15**

(51) Int Cl.:
***A61K 8/85*** *(2006.01)* ***A61K 8/87*** *(2006.01)*
***A61Q 5/06*** *(2006.01)*

(21) Numéro de dépôt: **05292219.2**

(22) Date de dépôt: **21.10.2005**

(54) **Composition cosmétique comprenant un polyester sulfonique et un polyuréthane**

Kosmetische Zusammensetzung enthaltend einen sulfonierten Polyester und einen Polyurethan

Cosmetic composition comprising a sulfonic polyester and a polyurethane

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.10.2004 FR 0452471**

(43) Date de publication de la demande:
**03.05.2006 Bulletin 2006/18**

(73) Titulaire: **L'ORÉAL**
**75008 Paris (FR)**

(72) Inventeurs:
- **Laurent, Ludivine**
**92400 Courbevoie (FR)**
- **Benabdillah, Katarina**
**92110 Clichy (FR)**

(74) Mandataire: **Bourdeau, Françoise**
**L'OREAL - D.I.P.I.**
**25-29 Quai Aulagnier**
**92600 Asnières (FR)**

(56) Documents cités:
**DE-A1- 19 928 772**
**FR-A- 2 783 163**
**FR-A- 2 846 884**

EP 1 652 510 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente demande a pour objet une composition cosmétique comprenant, dans un milieu cosmétiquement acceptable, au moins un polyester sulfonique dispersible dans l'eau et au moins un polyuréthane de haut poids moléculaire, ainsi que les procédés mettant en oeuvre cette composition cosmétique et les utilisations de cette composition cosmétique pour fixer la coiffure.

Les compositions cosmétiques pour la mise en forme et/ou le maintien de la coiffure peuvent être des compositions à pulvériser essentiellement constituées d'une solution le plus souvent alcoolique et d'un ou plusieurs composants, appelés composants fixants, qui sont généralement des résines polymères dont la fonction est de former des soudures entre les cheveux ou de gainer les cheveux. Ces composants fixants sont souvent formulés en mélange avec divers adjuvants cosmétiques.

Ces compositions cosmétiques sont généralement conditionnées soit dans un flacon pompe, soit dans un récipient aérosol approprié mis sous pression à l'aide d'un agent propulseur, le système aérosol contient alors d'une part une phase liquide (ou jus) et d'autre part d'un agent propulseur.

On peut également utiliser des compositions de coiffage sous forme de gels, de crèmes, de mousses.

Une fois appliquée sur les cheveux, les composés fixants doivent permettre la fixation de la chevelure

Or les polymères couramment utilisés en tant qu'agents fixants dans les compositions de coiffage ne permettent pas de conserver la mise en forme de la coiffure lorsque celle-ci est mise au contact de l'eau liquide pendant une durée prolongée telle que par exemple une mise en contact avec la pluie, la transpiration, ou lors des bains : bains de mer ou bains en piscine...

De manière surprenante est avantageuse, la demanderesse a découvert que l'utilisation d'une composition cosmétique, comprenant, dans un milieu cosmétiquement acceptable, au moins un polyester sulfonique dispersible dans l'eau et au moins un polyuréthane de haut poids moléculaire, permet de fixer et de mettre en forme les coiffures et aussi de conserver la mise en forme des coiffures lorsque celles-ci sont mise au contact de l'eau liquide pendant une durée prolongée.

Au sens de la présente demande, on nomme ce phénomène de « résistance à l'eau ».

Par « durée prolongée » au sens de la présente demande, on entend une mise en contact avec l'eau d'une durée minimale d'une minute, de préférence 10 minutes et de manière encore plus préférée 20 minutes.

Les compositions de l'invention permettent également d'obtenir un coiffage résistant à l'humidité de l'air.

Les compositions selon la présente invention permettent une bonne fixation et un bon maintien des cheveux c'est-à-dire un effet coiffant qui persiste tout au long de la journée, voire plusieurs jours, qui présente une bonne résistance à l'eau, notamment une bonne résistance aux bains répétés, ces compositions présentent aussi l'avantage de s'éliminer au shampooing.

Ces compositions permettent aussi de conférer de bonnes propriétés cosmétiques.

La présente invention a pour objet une composition cosmétique comprenant, dans un milieu cosmétiquement acceptable, au moins un polyester sulfonique dispersible dans l'eau et au moins polyuréthane spécifique.

Un autre objet de la présente invention consiste en un procédé pour la mise en forme ou le maintien de la coiffure dans lequel la composition cosmétique selon l'invention est mise en oeuvre.

Un troisième objet de l'invention concerne l'utilisation d'une composition cosmétique comprenant, dans un milieu cosmétiquement acceptable, au moins un polyester sulfonique dispersible dans l'eau et au moins polyuréthane spécifique, en tant que composition coiffante pour la fixation et le maintien des cheveux en particulier lorsqu'ils sont mis au contact de l'eau liquide pendant une durée prolongée c'est-à-dire en cas de pluie, de transpiration, lors de bains : tels que les bains de mer ou en piscine.

Selon ce troisième objet de l'invention, l'utilisation de la composition permet l'obtention d'une coiffure (mise en forme des cheveux) résistant à l'eau.

La composition cosmétique selon l'invention peut se présenter sous toutes les formes : lotions, sprays, mousses, gels, crèmes...

D'autres objets, caractéristiques, aspects et avantages de l'invention apparaîtront encore plus clairement à la lecture de la description et des exemples qui suivent.

Par « composition cosmétique coiffante » au sens de la présente demande, on entend une composition pour la mise en forme ou le maintien de la coiffure.

Le milieu cosmétiquement acceptable utilisé dans les compositions selon la présente demande est un milieu aqueux ou un milieu eau-solvant(s) organique(s).

Les solvants organiques utilisés dans les compositions selon la présente invention peuvent être les alcools inférieurs en $C_1$-$C_4$ comme l'éthanol, l'isopropanol, le tertiobutanol, le n-butanol, ou des polyols ou éthers de polyols. De préférence, le solvant utilisé est l'éthanol ou le propylène glycol ou le glycérol.

La concentration en solvant(s) dans les compositions selon la présente invention est comprise entre 0 et 70%, préférence entre 0 et 50% et de manière encore plus préférée entre 0 et 15% en poids par rapport au poids total de la

composition.

De préférence, la composition ne contient pas d'alcool en $C_1$-$C_4$.

À titre de solvant organique additionnel utilisable dans les compositions selon la présente invention, on compte les polyols comme le propylèneglycol, les éthers de polyols et leurs mélanges.

La concentration en solvant organique additionnel dans les compositions selon la présente invention est comprise entre 0 et 70%, de préférence entre 0 et 50% en poids par rapport au poids total de la composition, plus préférentiellement entre 0 et 15 %.

La composition selon l'invention comprend un polyester sulfonique dispersible dans l'eau.

Le polyester sulfonique est linéaire.

Par polyester sulfonique hydrodispersible, on entend tout polyester sulfonique présentant une aptitude à former une dispersion, c'est-à-dire un système biphasique où la première phase est formée de particules finement divisées et distribuées uniformément dans la seconde phase qui est la phase continue.

Les polyesters sulfoniques linéaires dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse (Tg ) de ces polyesters sulfoniques linéaires est généralement comprise dans l'intervalle allant de 10 °C à 100 °C. De préférence, la Tg du ou des polyesters utilisés est supérieure ou égale à 50°C.

La température de transition vitreuse (Tg) est mesurée par analyse enthalpique différentielle (DSC, *differential scanning calorimetry*) selon la norme ASTM D3418-97.

Les polyesters sulfoniques utilisés dans l'invention sont obtenus à partir d'acide isophtalique, de sel de sodium de l'acide sulfoisophtalique, de diéthylèneglycol et de 1,4-cyclohexaneméthanol.

A titre d'exemples de polyester sulfonique linéaires, on peut notamment citer ceux connus sous le nom INCI Diglycol/CHDM/Isophtalates/SIP, et vendus sous les dénominations commerciales"Eastman AQ polymer" (AQ35S, AQ38S, AQ55S, AQ48 Ultra) par la société Eastman Chemical.

La concentration en polyester(s) sulfonique(s) dispersible(s) dans l'eau utilisé(s) dans les compositions selon la présente invention est comprise entre 0,1 et 40%, de préférence entre 1 et 30% et de manière encore plus préférée entre 5 et 25% en poids par rapport au poids total de la composition.

## POLYURETHANES

Les polyuréthanes sont notamment présents dans la composition selon l'invention, en une quantité de 0,05 à 40 % en poids, de préférence de 0,1 à 20 % en poids, et mieux encore de 1 à 8 % en poids par rapport au poids total de la composition de traitement cosmétique des matières kératiniques.

Le polyuréthane de l'invention est l'Avalure UR-450 commercialisé par la Société Novéon, lequel est un copolymère anionique formé de PPG-17 (polypropylène glycol de nombre de motifs n=17)/IPDI (isophorone diisocyanate)/DMPA (diméthylol propionic acid). En milieu aqueux, il se présente sous la forme d'une dispersion. Son poids moléculaire est 1 830 000 g/mol.

## COMPOSITION

Les compositions selon la présente demande peuvent également contenir un ou plusieurs adjuvants cosmétiques additionnels tels que ceux mentionnés ci-dessous.

Tous les polymères fixants additionnels anioniques, cationiques, amphotères, non ioniques et leurs mélanges utilisés dans la technique peuvent être utilisés dans les compositions selon la présente demande.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule :

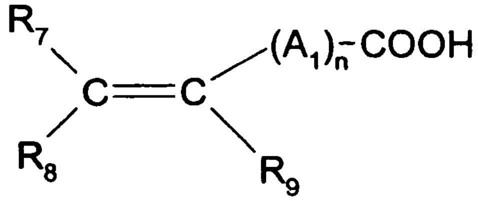

(I)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, $R_7$ désigne un atome d'hydrogène, un groupement phényle ou benzyle, $R_8$ désigne un atome d'hydrogène, un groupement alkyle inférieur ou carboxyle, $R_9$ désigne un atome d'hydrogène, un groupement alkyle inférieur, un groupement $-CH_2-COOH$, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques sont de préférence choisis parmi les homopolymères ou copolymères d'acide acrylique et méthacrylique ou leurs sels, les copolymères d'acide crotonique, les copolymères d'acides ou d'anhydrides carboxyliques monoinsaturés en $C_4$-$C_8$, les polyacrylamides à groupements carboxylates, les homopolymères ou copolymères à groupes sulfoniques, les polyuréthanes anioniques, et les polymères siliconés greffés anioniques.

Les polymères fixants anioniques à groupements carboxyliques préférés selon l'invention sont :

A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;

B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^os 75370 et 75371 ou proposés sous la dénomination QUADRAMER par la société AMERICAN CYANAMID. On peut également citer les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF ;

On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL;

C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique $\alpha$- ou $\beta$-cyclique. De tels polymères sont décrits entre autres dans les brevets français n^os 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;

D) Les copolymères d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^os 2 047 398, 2 723 248, 2 102 113, le brevet GB n° 839 805. Des produits commerciaux sont notamment ceux vendus sous les dénominations GANTREZ® AN ou ES par la société ISP ;
- les copolymères comprenant (i) un ou plusieurs motifs anhydrides maléique, citraconique, itaconique et (ii) un ou plusieurs monomères choisis parmi les esters allyliques ou méthallyliques comportant éventuellement un

ou plusieurs groupements acrylamide, méthacrylamide, α-oléfine, esters acryliques ou méthacryliques, acides acrylique ou méthacrylique ou vinylpyrrolidone dans leur chaîne,

les fonctions anhydrides de ces copolymères étant éventuellement monoestérifiées ou monoamidifiées.
Ces polymères sont par exemple décrits dans les brevets français n$^{os}$ 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.
Ces polymères peuvent être notamment choisis parmi :

- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan® 500 et Flexan® 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.
Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination Fixate G-100 par la société NOVEON.
Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.
Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.
De préférence, les polymères fixants cationiques sont choisis parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.
Parmi ces polymères, on peut citer plus particulièrement les polymères cationiques suivants:

(1) les homopolymères ou copolymères dérivés d'esters ou d'amides acryliques ou méthacryliques et comportant au moins un des motifs de formules suivantes:

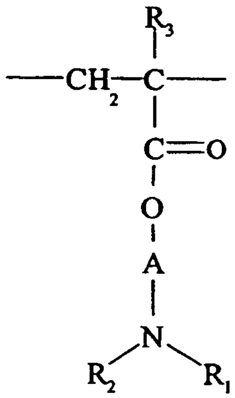

(A)

,

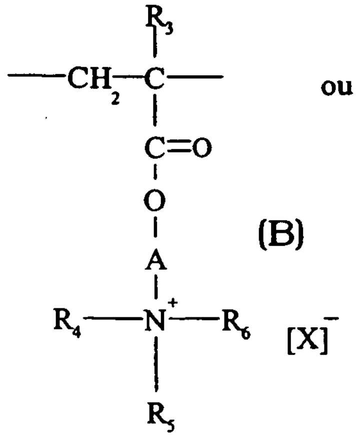

(B)

ou

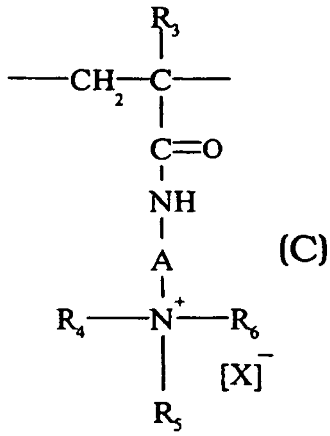

(C)

dans lesquelles:

$R_3$ désigne un atome d'hydrogène ou un radical $CH_3$;
A est un groupe alkyle linéaire ou ramifié comportant de 1 à 6 atomes de carbone ou un groupe hydroxyalkyle comportant de 1 à 4 atomes de carbone ;
$R_4$, $R_5$, $R_6$, identiques ou différents, représentent un groupe alkyle ayant de 1 à 18 atomes de carbone ou un radical benzyle;
$R_1$ et $R_2$, identiques ou différents, représentent chacun un atome hydrogène ou un groupe alkyle ayant de 1 à 6 atomes de carbone;
X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.
Ainsi, parmi ces copolymères de la famille (1), on peut citer :

- les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium tel que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
- les polymères à chaîne grasse et à motif vinylpyrrolidone, tels que les produits vendus sous la dénomination Stylèze W20 et Stylèze W 10 par la société ISP,
- les terpolymères méthacrylate de diméthylaminoéthyle/vinylcaprolactame/vinylpyrrolidone tel que le produit commercialisé sous la dénomination GAFFIX VC 713 par la société ISP, et
- et les copolymères vinylpyrrolidone/méthacrylamide de diméthylaminopropyle quaternisés tels que les produits commercialisés sous la dénomination "GAFQUAT® HS 100" par la société ISP.

(2) les polysaccharides cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13

S, JAGUAR C 15, JAGUAR C 17 par la société MEYHALL.

(3) les copolymères quaternaires de vinylpyrrolidone et de vinylimidazole ;

(4) les chitosanes ou leurs sels; les sels utilisables sont en particulier les acétate, lactate, glutamate, gluconate ou le pyrrolidone-carboxylate de chitosane . Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.

(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyldiallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-$\alpha,\beta$-dicarboxylique dont l'un des groupements carboxyliques a été amené à réagir avec une polyamine comportant un ou plusieurs groupements amines primaires ou secondaires.

De préférence, les polymères fixants amphotères sont choisis parmi les copolymères à motifs vinyliques acides et à motifs vinyliques basiques, les polyaminoamides réticulés et acylés, les polymères à motifs zwittérioniques, les polymères dérivés du chitosane, les copolymères alkyl($C_1$-$C_5$)vinyléther/anhydride maléique modifiés, les polyuréthanes amphotères et les polymères siliconés greffés amphotères.

Les polymères fixants amphotères répondant à la définition donnée ci-dessus plus particulièrement préférés sont choisis parmi les polymères suivants :

(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.

(2) les polymères comportant des motifs dérivant :

a) d'au moins un monomère choisi parmi les acrylamides ou les méthacrylamides substitués sur l'atome d'azote par un groupe alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique, ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.

(3) les polyaminoamides réticulés et acylés partiellement ou totalement dérivant de polyaminoamides de formule générale :

$$-[CO-R_{10}-CO-Z]- \qquad (II)$$

dans laquelle $R_{10}$ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :

a) dans les proportions de 60 à 100 % en moles, le groupe

$$-NH-[(CH_2)_x-NH]_p-$$

où x=2 et p=2 ou 3, ou bien x=3 et p=2

ce groupe dérivant de la diéthylène-triamine, de la triéthylène-tétraamine ou de la dipropylène-triamine;

b) dans les proportions de 0 à 40 % en moles, le groupe (IV) ci-dessus, dans lequel x=2 et p=1 et qui dérive de l'éthylène-diamine, ou le groupe dérivant de la pipérazine :

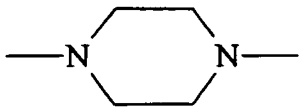

c) dans les proportions de 0 à 20 % en moles, le groupe $-NH-(CH_2)_6-NH-$ dérivant de l'hexaméthylènediamine,

ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.

Les alcane-sultones utilisées dans l'acylation sont de préférence la propane- ou la butane-sultone, les sels des agents d'acylation sont de préférence les sels de sodium ou de potassium.

(4) les polymères comportant des motifs zwittérioniques de formule :

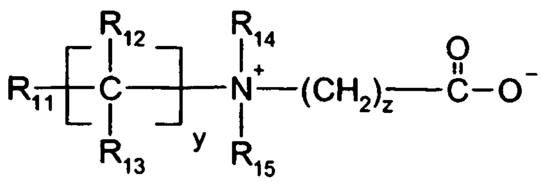

(IV)

dans laquelle $R_{11}$ désigne un groupement insaturé polymérisable tel qu'un groupement acrylate, méthacrylate, acrylamide ou méthacrylamide, y et z représentent un nombre entier de 1 à 3, $R_{12}$ et $R_{13}$ représentent un atome

d'hydrogène, un groupe méthyle, éthyle ou propyle, $R_{14}$ et $R_{15}$ représentent un atome d'hydrogène ou un groupe alkyle de telle façon que la somme des atomes de carbone dans $R_{14}$ et $R_{15}$ ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.

(5) les polymères dérivés du chitosane comportant des motifs monomères répondant aux formules suivantes :

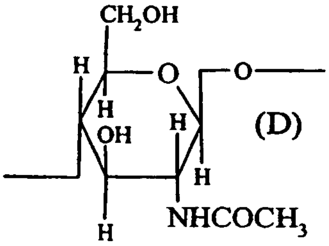
(D)

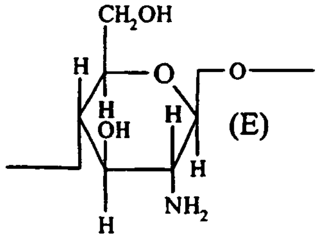
(E)

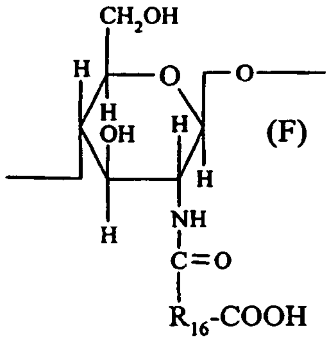
(F)

le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), $R_{16}$ représente un groupe de formule :

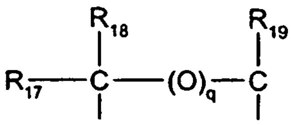

dans laquelle si q=0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes $R_{17}$, $R_{18}$ et $R_{19}$ étant dans ce cas un atome d'hydrogène ;

ou si q=1, $R_{17}$, $R_{18}$ et $R_{19}$ représentent chacun un atome d'hydrogène, ainsi que les sels formés par ces composés avec des bases ou des acides.

(6) Les polymères répondant à la formule générale (V) sont, par exemple, décrits dans le brevet français 1 400 366 :

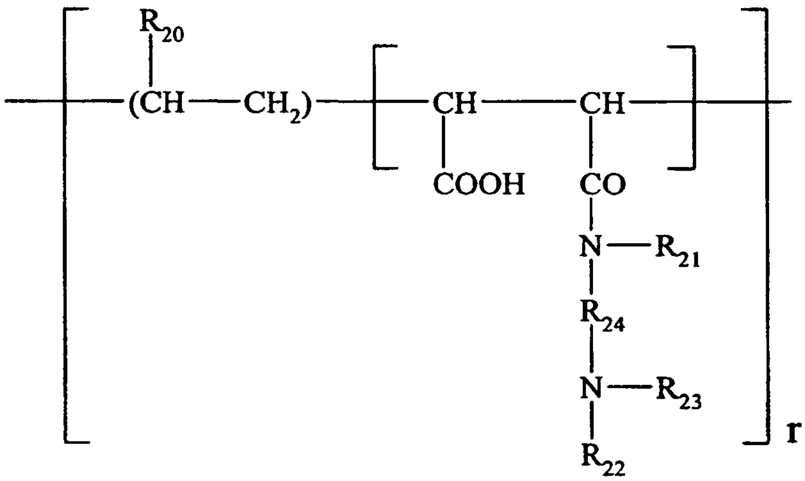
(V)

dans laquelle $R_{20}$ représente un atome d'hydrogène, un groupe $CH_3O$, $CH_3CH_2O$, phényle, $R_{21}$ désigne un atome d'hydrogène ou un groupe alkyle inférieur tel que méthyle, éthyle, $R_{22}$ désigne un atome d'hydrogène ou un groupe alkyle inférieur en $C_1$-$C_6$ tel que méthyle, éthyle, $R_{23}$ désigne un groupe alkyle inférieur en $C_1$-$C_6$ tel que méthyle, éthyle ou un groupe répondant à la formule : $-R_{24}-N(R_{22})_2$, $R_{24}$ représentant un groupement $-CH_2-CH_2-$, $-CH_2-CH_2-CH_2-$, $-CH_2-CH(CH_3)-$, $R_{22}$ ayant les significations mentionnées ci-dessus.

(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.

(8) Les polymères amphotères du type -D-X-D-X choisis parmi:

a) les polymères obtenus par action de l'acide chloracétique ou le chloracétate de sodium sur les composés comportant au moins un motif de formule :

-D-X-D-X-D- (VI)

où D désigne un groupe

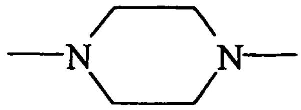

et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.

b) Les polymères de formule :

-D-X-D-X- (VI')

où D désigne un groupe

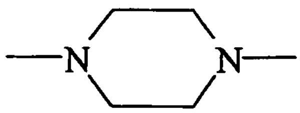

et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.

(9) les copolymères alkyl($C_1$-$C_5$)vinyléther/anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la

dénomination DIAFORMER Z301 par la société SANDOZ.

Les polymères fixants non ioniques utilisables selon la présente invention sont choisis, par exemple, parmi :

- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth) acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILTTH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :

a) de 50 à 90 % en poids d'acrylate de tertiobutyle,
b) de 0 à 40 % en poids d'acide acrylique,
c) de 5 à 40 % en poids d'un macromère siliconé de formule

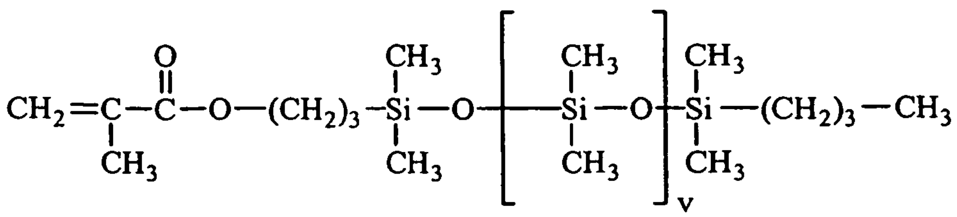

où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères

mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges de poids moléculaire inférieur à 400 000.

Comme polyuréthanes fixants additionnels convenant particulièrement bien dans la présente invention, on peut citer les produits commercialisés sous les dénominations LUVISET PUR® et LUVISET® Si PUR par la société BASF.

La concentration en polymère(s) fixant(s) additionnel(s) utilisé(s) dans les compositions selon la présente invention est comprise entre 0,1 et 20%, de préférence entre 0,5 et 10% en poids par rapport au poids total de la composition.

Les compositions selon la présente demande peuvent également contenir en tant qu'adjuvant cosmétique additionnel au moins un polymère épaississant additionnel encore appelé "agent d'ajustement de la rhéologie" différent des polyuréthanes.

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), les gommes de guar et ses dérivés tels que la gomme de guar hydroxypropylée, les gommes d'origine microbienne (gomme de xanthane, gomme de scléroglucane), les homopolymères réticulés d'acide acrylique ou d'acide acrylamidopropanesulfonique et les polymères associatifs.

Les polymères associatifs utilisables selon l'invention sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs utilisables selon l'invention peuvent être de type anionique, cationique, amphotère ou non ionique.

La concentration en polymère(s) épaississant(s) additionnel(s) est comprise entre 0,01 et 20%, de préférence entre 0,05 et 10% en poids par rapport au poids total de la composition

Les compositions selon la présente demande peuvent également contenir en tant qu'adjuvant cosmétique additionnel au moins un composé choisi parmi les silicones, les corps gras siliconés et les corps gras non siliconé.

Les silicones utilisables dans les compositions selon la présente invention peuvent être linéaires, cyclique, ramifiées ou non ramifiées, volatiles ou non volatiles. Elles peuvent être sous forme soluble, dispersée, micro-dispersée et se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :

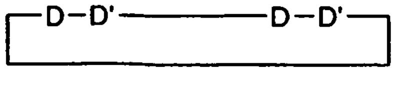

avec D :

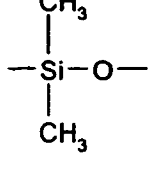

avec D' :

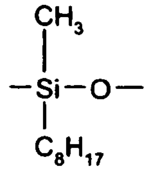

On peut également citer les mélanges de silicones cycliques avec des composés organiques dérivés du silicium,

tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à 5.10-6m2/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicone, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en C6-C24 tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C12)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "BABIL® S201" et "ABIL® S255".
- des groupements hydroxyacylamino, comme les polyorgano-siloxanes décrits dans la demande EP 342 834. On peut citer, par exemple, le produit Q2-8413 de la société DOW CORNING.

Les huiles de silicone utilisables dans les compositions selon l'invention sont des polyméthylsiloxanes volatiles ou non, à chaîne siliconée linéaire ou cyclique, liquides ou pâteuse à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, les polyméthyl-phénylsiloxanes ; et leurs mélanges.

Les gommes de silicone utilisables dans les compositions selon l'invention sont des polydiorganosiloxanes de masse moléculaire élevée, comprise entre 200 000 et 5 000 000 g/mol, utilisées seules ou en mélange dans un solvant choisi parmi les silicones volatiles, les huiles polydiméthylsiloxanes, les huiles polyméthylphénylsiloxanes, les huiles polydiphényldiméthylsiloxanes, les isoparaffines, le chlorure de méthylène, le pentane, les hydrocarbures ou leurs mélanges.

On utilise de préférence une gomme de silicone de poids moléculaire inférieur à 1 500000. Les gommes de silicone sont par exemple des polydiméthylsiloxanes, des polyphénylméthylsiloxanes, des poly(diphénylsiloxane diméthylsiloxanes), des poly(diméthylsiloxaneméthylvinylsiloxanes), des poly(diméthylsiloxanephénylméthylsiloxanes), des poly(diphénylsiloxane diméthylsiloxaneméthylvinylsiloxanes).

Ces gommes de silicones peuvent être terminées en bout de chaîne par des groupements triméthylsilyls ou diméthylhydroxysilyls.

Les résines de silicone utilisables dans les compositions selon l'invention sont des systèmes siloxaniques réticulés renfermant les unités $R_2SiO_{2/2}$, $RSiO_{3/2}$, $SiO_{4/2}$, dans lesquelles R représente un groupe hydrocarbonés possédant de 1 à 6 atomes de carbone ou un groupe phényle. Parmi ces produits, ceux particulièrement préférés sont ceux dans lesquelles R désigne un radical alkyl inférieur ($C_1$-$C_6$) ou un radical phényle.

Les corps gras non siliconés utilisables dans les compositions selon la présente invention sont toutes les huiles, cires, résines non siliconées organiques ou minérales, naturelles ou synthétiques.

Une huile, au sens de la présente invention, est un composé lipophile, liquide à température ambiante (environ 25 °C), à changement d'état solide/liquide réversible. Les huiles animales et végétales comprennent comme constituants essentiels des triesters du propane-1,2,3-triol.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme par exemple ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules $R^6COOR^7$ et $R^6OR^7$ dans laquelle $R^6$ représente le reste d'un acide gras comportant de 8 à 29 atomes de carbone, et $R^7$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle ; les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras fluides ayant de 8 à 26 atomes de carbone, comme l'octyldodécanol, le 2-butyloctanol, l'alcool oléique, l'alcool linoléique ou l'alcool linolénique ;
- les alcools gras alcoxylés et notamment éthoxylés tels que l'oleth-12 ;
- les huiles fluorées partiellement hydrocarbonées comme celles décrites dans le document JP-A-2 295912. Comme huiles fluorées, on peut citer aussi le perfluorométhylcyclopentane et le perfluoro-1,3- diméthylcyclohexane, par exemple vendus sous les dénominations de « FLUTEC PC1® » et « FLUTEC PC3® » par la Société BNFL Fluorochemicals; le perfluoro-1,2-diméthylcyclobutane ; les perfluoralcanes tels que le dodécafluoropentane et le tétradécafluorohexane, par exemple vendus sous les dénominations de « PF 5050® » et « PF 5060® » par la Société 3M, ou encore le bromoperfluorooctyle par exemple vendu sous la dénomination « FORALKYL® » par la Société Atochem ; le nanofluorométhoxybutane par exemple vendu sous la dénomination « MSX 4518® » par la Société 3M et le nanofluoroéthoxyisobutane ; les dérivés de perfluoromorpholine, tels que la 4-trifluorométhyl perfluoromorpholine par exemple vendue sous la dénomination « PF 5052® » par la Société 3M.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch, et leurs mélanges.

La concentration en composé(s) choisi(s) parmi les silicones, les corps gras siliconés et les corps gras non siliconé est comprise entre 0,01 et 20% et de préférence entre 0,05 et 10% en poids par rapport au poids total de la composition.

La composition coiffante selon l'invention peut contenir en outre au moins un additif choisi parmi les agents tensio-actifs non-ioniques, anioniques, cationiques et amphotères, les polymères non-ioniques, anioniques, cationiques et amphotères additionnels autres que les polymères fixants utilisés dans les compositions selon l'invention, les céramides et pseudo-céramides, les vitamines et pro-vitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

EPAISSISSANTS

On peut citer, par exemple, comme épaississant naturels convenant pour l'invention les gommes de xanthane, de scléroglucane, de gellane, de rhamsan, les alginates, la maltodextrine, l'amidon et ses dérivés, la gomme de karaya, la farine de caroube, les gommes de guar.

Les agents épaississants synthétiques selon l'invention sont avantageusement des polymères ou copolymères d'acide acrylique et/ou méthacrylique, comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate commercialisé par la société GUARDIAN sous la dénomination Lubragel ou encore le polyglycérylacrylate commercialisé sous la dénomination Hispagel par la société HISPANO CHIMICA.

Comme autres composés acryliques, on peut citer les copolymères d'acide acrylique ou méthacrylique comprenant au moins un motif acrylate d'alkyle en C1 à C30 et/ou un motif uréthanne éventuellement substitué par une chaîne grasse. On peut en particulier citer le Pemulen TR1 (Goodrich), le Viscophobe DB 1000 (Union Carbide) et les Acrysol 44 ou 46 (Rohm et Haas).

On peut encore utiliser comme agent épaississant les polyéthylèneglycols (PEG) et leurs dérivés.

On peut également utiliser avantageusement, en tant qu'agent épaississant, des polyacrylamides épaississants. Ceux-ci peuvent plus particulièrement être choisis parmi :

- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium éventuellement réticulés,
- les copolymères d'acrylamide et d'acide 2-acrylamido 2-méthyl propanesulfonique partiellement ou totalement neutralisés éventuellement réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de $10^{-4}$ à $4.10^{-4}$ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en $C_{12}$-$C_{13}$, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (environ 50/50 en poids) sous forme de dispersion contenant 50 % en poids dudit copolymère dans de l'huile minérale. Cette dispersion est commercialisée sous le nom de "SALCARE SC92" par la Société ALLIED COLLOIDS.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

TENSIOACTIFS

Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels d'alcalinoterreux (de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

(ii) Tensioactif(s) non ionique(s) :

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides,

les dérivés de N-alkyl glucamine, les oxydes d'amines tels que les oxydes d'alkyl ($C_{10}$-$C_{14}$) amines ou les oxydes de N-acylaminopropylmorpholine.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL®, tels que décrits dans les brevets US-2 528 378 et US-2 781 354 et de structures :

$$R_2\text{-}CONHCH_2CH_2\text{-}N(R_3)(R_4)(CH_2COO\text{-}) \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_{2'}\text{-}CONHCH_2CH_2\text{-}N(B)(C) \qquad (3)$$

dans laquelle :

B représente $-CH_2CH_2OX'$, C représente $-(CH_2)_z-Y'$, avec z = 1 ou 2,
X' désigne le groupement $-CH_2CH_2-COOH$ ou un atome d'hydrogène
Y' désigne -COOH ou le radical $-CH_2$ - CHOH - $SO_3H$
$R_2$, désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique ou non ionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium oxyéthylénés à 2,2 moles d'oxyde d'éthylène, le cocoyl iséthionate de sodium et l'alphaoléfine($C_{14}$-$C_{16}$) sulfonate de sodium et leurs mélange avec

- soit un agent tensioactif amphotère tel que les dérivés d'amine dénommés disodiumcocoamphodipropionate ou sodiumcocoamphopropionate commercialisés notamment par la société RHODIA sous la dénomination commerciale "MIRANOL® C2M CONC" en solution aqueuse à 38 % de matière active ou sous la dénomination MIRANOL® C32;

- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidobétaïnes telles que la TEGOBETAINE® F50 commercialisée par la société GLODSCHMIDT.

Tensioactifs cationiques

La composition selon l'invention comprend un ou plusieurs tensioactifs cationiques bien connus en soi, tels

que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :

- ceux qui présentent la formule générale (VI) suivante :

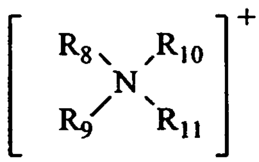

$X^-$ (VI)

dans laquelle les radicaux $R_8$ à $R_{11}$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène ($C_2$-$C_6$), alkylamide, alkyl($C_{12}$-$C_{22}$)amidoalkyle($C_2$-$C_6$), alkyl($C_{12}$-$C_{22}$)acétate, hydroxyalkyle, comportant environ de 1 à 30 atomes de carbone ; X est un anion choisi dans le groupe des halogénures, phosphates, acétates, lactates, alkyl($C_2$-$C_6$)sulfates, alkyl- ou alkylaryl-sulfonates ;

- les sels d'ammonium quaternaire de l'imidazoline, comme par exemple ceux de formule (VII) suivante :

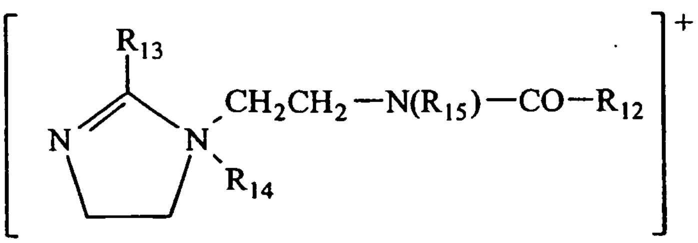

$X^-$ (VII)

dans laquelle $R_{12}$ représente un radical alcényle ou alkyle comportant de 8 à 30 atomes de carbone, par exemple dérivés des acides gras du suif, $R_{13}$ représente un atome d'hydrogène, un radical alkyle en $C_1$-$C_4$ ou un radical alcényle ou alkyle comportant de 8 à 30 atomes de carbone, $R_{14}$ représente un radical alkyle en $C_1$-$C_4$, $R_{15}$ représente un atome d'hydrogène, un radical alkyle en $C_1$-$C_4$, $X^-$ est un anion choisi dans le groupe des halogénures, phosphates, acétates, lactates, alkylsulfates, alkyl- ou alkylaryl-sulfonates. De préférence, $R_{12}$ et $R_{13}$ désignent un mélange de radicaux alcényle ou alkyle comportant de 12 à 21 atomes de carbone, par exemple dérivés des acides gras du suif, $R_{14}$ désigne un radical méthyle, $R_{15}$ désigne un atome d'hydrogène. Un tel produit est par exemple commercialisé sous la dénomination REWOQUAT® W 75 par la société REWO ;

- les sels de diammonium quaternaire de formule (VIII) :

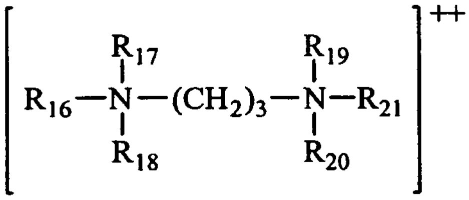

$2X^-$ (VIII)

dans laquelle $R_{16}$ désigne un radical aliphatique comportant environ de 16 à 30 atomes de carbone, $R_{17}$, $R_{18}$, $R_{19}$, $R_{20}$ et $R_{21}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.

De tels sels de diammonium quaternaire comprennent notamment le dichlorure de propanesuif diammonium ;

- les sels d'ammonium quaternaire contenant au moins une fonction ester, tels que ceux de formule (IX) suivante :

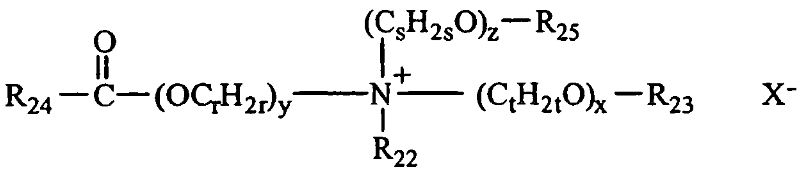 (IX)

dans laquelle :

$R_{22}$ est choisi parmi les radicaux alkyles en $C_1$-$C_6$ et les radicaux hydroxyalkyles ou dihydroxyalkyles en $C_1$-$C_6$ ;
$R_{23}$ est choisi parmi :

- le radical

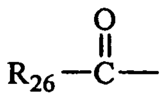

- les radicaux $R_{27}$ hydrocarbonés en $C_1$-$C_{22}$, linéaires ou ramifiés, saturés ou insaturés,
- l'atome d'hydrogène,

$R_{25}$ est choisi parmi :

- le radical

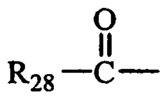

- les radicaux $R_{29}$ hydrocarbonés en $C_1$-$C_6$, linéaires ou ramifiés, saturés ou insaturés,
- l'atome d'hydrogène,

$R_{24}$, $R_{26}$ et $R_{28}$, identiques ou différents, sont choisis parmi les radicaux hydrocarbonés en $C_7$-$C_{21}$, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
$X^-$ est un anion simple ou complexe, organique ou inorganique ;
sous réserve que la somme x + y + z vaut de 1 à 15, que lorsque x vaut 0 alors $R_{23}$ désigne $R_{27}$ et que lorsque z vaut 0 alors $R_{25}$ désigne $R_{29}$.

Les radicaux alkyles $R_{22}$ peuvent être linéaires ou ramifiés et plus particulièrement linéaires.
De préférence $R_{22}$ désigne un radical méthyle, éthyle, hydroxyéthyle ou dihydroxypropyle, et plus particulièrement un radical méthyle ou éthyle.
Avantageusement, la somme x + y + z vaut de 1 à 10.
Lorsque $R_{23}$ est un radical $R_{27}$ hydrocarboné, il peut être long et avoir de 12 à 22 atomes de carbone, ou court et avoir de 1 à 3 atomes de carbone.
Lorsque $R_{25}$ est un radical $R_{29}$ hydrocarboné, il a de préférence 1 à 3 atomes de carbone.
Avantageusement, $R_{24}$, $R_{26}$ et $R_{28}$, identiques ou différents, sont choisis parmi les radicaux hydrocarbonés

en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion $X^-$ est encore plus particulièrement le chlorure ou le méthylsulfate.

On utilise plus particulièrement dans la composition selon l'invention, les sels d'ammonium de formule (IX) dans laquelle :

- $R_{22}$ désigne un radical méthyle ou éthyle,
- x et y sont égaux à 1 ;
- z est égal à 0 ou 1 ;
- r, s et t sont égaux à 2 ;
- $R_{23}$ est choisi parmi :

  - le radical

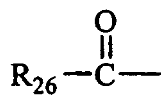

  - les radicaux méthyle, éthyle ou hydrocarbonés en $C_{14}$-$C_{22}$,
  - l'atome d'hydrogène ;

- $R_{25}$ est choisi parmi :

  - le radical

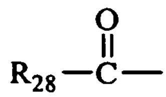

  - l'atome d'hydrogène ;

- $R_{24}$, $R_{26}$ et $R_{28}$, identiques ou différents, sont choisis parmi les radicaux hydrocarbonés en $C_{13}$-$C_{17}$, linéaires ou ramifiés, saturés ou insaturés, et de préférence parmi les radicaux alkyles et alcényles en $C_{13}$-$C_{17}$, linéaires ou ramifiés, saturés ou insaturés.

Avantageusement, les radicaux hydrocarbonés sont linéaires. On peut citer par exemple les composés de formule (IX) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART® par la société

HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium et le chlorure de palmitylamidopropyltriméthylammonium.

La composition selon l'invention comprend de préférence le ou les tensioactifs en une quantité comprise entre 0,1 et 10 % en poids, mieux encore entre 0,5 et 8 % en poids, et encore plus préférentiellement entre 1 et 5 % en poids par rapport au poids total de la composition.

Avantageusement, on choisit, comme tensioactif, un tensioactif amphotère ou non ionique.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

L'exemple qui suit illustre la présente invention et ne doit en aucune manière être considéré comme limitant l'invention.

Nous préparons une laque selon l'invention :

| | |
|---|---|
| a. Avalure UR 450 (Noveon) | 1% m.a. |
| b. Eastman AQ 55S | 1%m.a. |
| c. Q2-5220 (Dow Corning) | 0.5% m.a. |
| d. eau | 53.5% m.a. |
| e. DME | 40%m.a. |

Nous préparons un spray fixant du type eau/air dans un récipient à poche sous pression selon l'invention. Le jus contient :

| | |
|---|---|
| a. Avalure UR 450 (Noveon) | 1% m.a. |
| b. Eastman AQ 55S | 1%m.a. |
| c. eau | 98% m.a. |

Nous préparons une mousse selon l'invention :

| | |
|---|---|
| a. Avalure UR 450 (Noveon) | 3% m.a. |
| b. Monolaurate de sorbitane oxyéthyléné (20 OE) | 0.2% m.a. |
| c. eau | 88.8% m.a. |
| d. isobutane/propane (85/15) | 5% m.a. |
| i. Eastman AQ 55S | 3%m.a. |

Nous préparons un gel de coiffage selon l'invention :

| | |
|---|---|
| a. Avalure UR 450 (Noveon) | 5% m.a. |
| b. Eastman AQ 55S | 5%m.a. |

(suite)

| | |
|---|---|
| c. Jaguar HP105 (Rhodia) | 1% m.a. |
| d. Q2-5220 (Dow Corning) | 1% m.a. |
| e. eau | qsp 100% m.a. |

Nous préparons un spray fixant non aérosol selon l'invention :

| | |
|---|---|
| a. Avalure UR 450 (Noveon) | 3% m.a. |
| b. Eastman AQ 55S | 3% m.a. |
| c. eau | 94% m.a. |

EASTMANN AQ 55 S commercialisé par EASTMANN est un copolymère de diéthylèneglycol / 1,4-cyclohexane-diméthanol/isophtalate/ sulfoisophtalate, c'est un polyester sulfonique linéaire dispersible dans l'eau

Protocole opératoire :

- Sur mèche de cheveux naturels de 2,7g et de longueur 27 cm, appliquer 2 g de la formule à tester.
- Enrouler la mèche traitée autour d'un bigoudi de diamètre 1 cm, pour lui donner une forme.
- Laisser sécher le produit à l'air, puis défaire délicatement la mèche du bigoudi.
- Les mèches ainsi mises en forme sont ensuite immergées dans un bain d'eau salée (3% NaCl) de volume 8 litres à température ambiante, sous agitation magnétique à 100 tours/minute.
- Mesurer la longueur de la mèche au cours du temps afin d'évaluer le maintien de la mise en forme.

Mesures du maintien de la mise en forme :

$$\text{Maintien de la mise en forme en \% : } (L_i - L)/(L_i - L_0)*100$$

L : Longueur de la mèche bouclée au temps t
$L_0$ : Longueur de la mèche bouclée après mise en forme et retrait du bigoudi
$L_i$ : Longueur de la mèche avant mise en forme sur bigoudi

| Temps d'immersion | Maintien de la mise en forme en % |
|---|---|
| 0 | 100 |
| 35 secondes | 100 |
| 40 secondes | 100 |
| 10 minutes | 100 |

## Revendications

1. Composition cosmétique comprenant, dans un milieu cosmétiquement acceptable, au moins un polyester sulfonique dispersible dans l'eau et au moins un polyuréthane,

   - le polyester sulfonique étant linéaire et obtenu à partir d'acide isophtalique, de sel de sodium de l'acide sulfoisophtalique, de diéthylèneglycol et de 1,4-cyclohexaneméthanol, et
   - le polyuréthane étant un copolymère anionique formé de PPG-17/IPDI/DMPA, se présentant, en milieu aqueux, sous la forme d'une dispersion et son poids moléculaire étant 1 830 000 g/mol.

**2.** Composition cosmétique selon l'une des revendications précédentes telle que la concentration en polyester(s) sulfonique(s) dispersible(s) dans l'eau est comprise entre 0,1 et 40%, de préférence entre 1 et 30% et de manière encore plus préférée entre 5 et 25% en poids par rapport au poids total de la composition.

**3.** Composition cosmétique selon l'une des revendications précédentes telle que la concentration en polyuréthane(s) est comprise entre 0,01 et 40%, de préférence entre 0,05 et 20% et de manière encore plus préférée entre 0,1 et 10% en poids par rapport au poids total de la composition.

**4.** Composition cosmétique selon l'une des revendications précédentes telle qu'elle comprend un polymère fixant additionnel choisi parmi les polymères fixants anioniques, cationiques, amphotères, non-ioniques, ou leurs mélanges.

**5.** Composition selon la revendication 4, **caractérisée en ce que** le polymère fixant cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

**6.** Composition selon la revendication 4, **caractérisée en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en $C_4$-$C_8$, les polyacrylamides à groupements carboxylates, les homopolymères ou copolymères à groupes sulfoniques, les polyuréthanes anioniques, et les polymères siliconés greffés anioniques.

**7.** Composition selon la revendication 4, **caractérisée en ce que** le polymère fixant amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl($C_1$-$C_5$)vinyléther/anhydride maléique modifiés, les polyuréthanes amphotères et les polymères siliconés greffés amphotères.

**8.** Composition selon la revendication 4, **caractérisée en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

**9.** Composition cosmétique selon l'une des revendications 4 à 8 telle que la concentration en polymère(s) fixant(s) additionnel(s) est comprise entre 0,1 et 20%, de préférence entre 0,5 et 10% en poids par rapport au poids total de la composition.

**10.** Composition cosmétique selon l'une des revendications 4 à 8, telle que les solvants organiques utilisés dans les compositions selon la présente invention peuvent être les alcools inférieurs en $C_1$-$C_4$ comme l'éthanol, l'isopropanol, le tertiobutanol, le n-butanol, ou des polyols ou éthers de polyols.

**11.** Composition cosmétique selon l'une des revendications précédentes telle qu'elle comprend au moins un composé choisi parmi les silicones, les corps gras siliconés et les corps gras non siliconé.

**12.** Composition cosmétique selon la revendication 11 telle que la concentration en composé(s) choisi(s) parmi les silicones, les corps gras siliconés et les corps gras non siliconé est comprise entre 0,01% et 20%, de préférence entre 0,05% et 10% en poids par rapport au poids total de la composition.

**13.** Composition cosmétique selon l'une des revendications précédentes telle qu'elle contient au moins un additif choisi parmi les agents tensio-actifs non-ioniques, anioniques, cationiques et amphotères, les polymères non-ioniques, anioniques, cationiques et amphotères additionnels, les céramides et pseudo-céramides, les vitamines et provitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

**14.** Composition cosmétique selon l'une quelconque des revendications précédentes, **caractérisée en ce qu'**elle ne contient pas d'alcool en $C_1$-$C_4$.

**15.** Procédé pour la mise en forme ou le maintien de la coiffure dans lequel la composition cosmétique selon l'une des revendications 1 à 14 est mise en oeuvre.

**16.** Utilisation de la composition cosmétique selon l'une des revendications 1 à 14 pour la fixation et le maintien des cheveux.

**17.** Utilisation de la composition selon la revendication 16, pour l'obtention d'une coiffure résistant à l'eau.

## Claims

**1.** Cosmetic composition comprising in a cosmetically acceptable medium at least one water-dispersible sulphonic polyester and at least one polyurethane,

- the sulphonic polyester being linear and obtained from isophthalic acid, the sodium salt of sulphoisophthalic acid, diethylene glycol and 1,4-cyclohexanedimethanol, and
- the polyurethene being an anionic copolymer formed from PPG-17/IPDI/DMPA; being, in an aqueous medium, in the form of a dispersion; and its molecular weight being 1 830 000 g/mol.

**2.** Cosmetic composition according to Claim 1, such that the concentration of water-dispersible sulphonic polyester(s) is between 0.1% and 40%, preferably between 1% and 30% and more preferably between 5% and 25% by weight relative to the total weight of the composition.

**3.** Cosmetic composition according to either of the preceding claims, such that the concentration of polyurethane(s) is between 0.01% and 40%, preferably between 0.05% and 20% and more preferably between 0.1% and 10% by weight relative to the total weight of the composition.

**4.** Cosmetic composition according to one of the preceding claims, such that it comprises an additional fixative polymer selected from anionic, cationic, amphoteric and nonionic fixative polymers or mixtures thereof.

**5.** Composition according to Claim 4, **characterized in that** the cationic fixative polymer is selected from homopolymers or copolymers of acrylic or methacrylic esters or amides containing amino functions, cationic polysaccharides, quaternary vinylpyrrolidone-vinylimidazole copolymers, and chitosans.

**6.** Composition according to Claim 4, **characterized in that** the anionic fixative polymer is selected from homopolymers or copolymers of acrylic and methacrylic acid or their salts, copolymers of crotonic acid, copolymers of $C_4$-$C_8$ monounsaturated carboxylic acids or anhyrides, polyacrylamides containing carboxylate groups, homopolymers or copolymers containing sulphonic groups, anionic polyurethanes, and grafted anionic silicone polymers.

**7.** Composition according to Claim 4, **characterized in that** the amphoteric fixative polymer is selected from copolymers containing acidic vinyl units and containing basic vinyl units, crosslinked and acylated polyaminoamides, polymers containing zwitterionic units, polymers derived from chitosan, modified ($C_1$-$C_5$)alkyl vinyl ether/maleic anhydride copolymers, amphoteric polyurethanes, and grafted amphoteric silicone polymers.

**8.** Composition according to Claim 4, **characterized in that** the nonionic fixative polymer is selected from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers, nonionic polyurethanes, and grafted nonionic silicone polymers.

**9.** Cosmetic composition according to one of Claims 4 to 8, such that the concentration of additional fixative polymer (s) is between 0.1% and 20%, preferably between 0.5% and 10% by weight relative to the total weight of the composition.

**10.** Cosmetic composition according to one of Claims 4 to 8, such that the organic solvents used in the compositions according to the present invention may be $C_1$-$C_4$ lower alcohols such as ethanol, isopropanol, tert-butanol, n-butanol,

or polyols or polyol ethers.

**11.** Cosmetic composition according to one of the preceding claims, such that it comprises at least one compound selected from silicones, silicone fatty substances and non-silicone fatty substances.

**12.** Cosmetic composition according to Claim 11, such that the concentration of compound(s) selected from silicones, silicone fatty substances and non-silicone fatty substances is between 0.01% and 20%, preferably between 0.05% and 10% by weight relative to the total weight of the composition.

**13.** Cosmetic composition according to one of the preceding claims, such that it comprises at least one additive selected from nonionic, anionic, cationic and amphoteric surfactants, additional nonionic, anionic, cationic and amphoteric polymers, ceramides and pseudoceramides, vitamins and provitamins, including panthenol, silicone or non-silicone water-soluble and lipid-soluble sunscreens, fillers and solid particles, for instance coloured or uncoloured mineral and organic pigments, nacreous agents and opacifiers, flakes, active particles, dyes, sequestrants, plasticizers, solubilizers, acidifying agents, alkalifying agents, neutralizing agents, organic and inorganic thickeners, antioxidants, hydroxy acids, penetrants, fragrances and preservatives.

**14.** Cosmetic composition according to any one of the preceding claims, **characterized in that** it contains no $C_1$-$C_4$ alcohol.

**15.** Method of shaping or holding the hairstyle, in which the cosmetic composition according to one of Claims 1 to 14 is employed.

**16.** Use of the cosmetic composition according to one of Claims 1 to 14 for fixing and holding the hair.

**17.** Use of the composition according to Claim 16, to obtain a water-resistant hairstyle.

**Patentansprüche**

**1.** Kosmetische Zusammensetzung, die in einem kosmetisch akzeptablen Medium mindestens einen in Wasser dispergierbaren Sulfopolyester und mindestens ein Polyurethan enthält,

- wobei der Sulfopolyester linear ist und ausgehend von Isophthalsäure, dem Natriumsalz von Sulfoisophthalsäure, Diethylenglycol und 1,4-Cyclohexanmethanol hergestellt wird, und
- das Polyurethan ein anionisches Copolymer ist, das aus PPG-17/IPDI/DMPA gebildet ist, in einem wässrigen Medium in Form einer Dispersion vorliegt und ein Molekulargewicht von 1 830 000 g/mol aufweist.

**2.** Kosmetische Zusammensetzung nach einem der vorhergehenden Ansprüche, die so ist, dass die Konzentration des in Wasser dispergierbaren Sulfopolyesters oder der in Wasser dispergierbaren Sulfopolyester im Bereich von 0,1 bis 40 %, vorzugsweise 1 bis 30 % und noch bevorzugter 5 bis 25 Gew.-%, bezogen auf das Gesamtgewicht der Zusammensetzung, liegt.

**3.** Kosmetische Zusammensetzung nach einem der vorhergehenden Ansprüche, wobei die Konzentration des Polyurethans oder der Polyurethane im Bereich von 0,01 bis 40 %, vorzugsweise 0,05 bis 20 % und noch bevorzugter 0,1 bis 10 Gew.-%, bezogen auf das Gesamtgewicht der Zusammensetzung, liegt.

**4.** Kosmetische Zusammensetzung nach einem der vorhergehenden Ansprüche, wobei sie ein ergänzendes fixierendes Polymer enthält, das unter den anionischen, kationischen, amphoteren, nichtionischen fixierenden Polymeren oder deren Gemischen ausgewählt ist.

**5.** Zusammensetzung nach Anspruch 4, **dadurch gekennzeichnet, dass** das kationische fixierende Polymer unter den Homopolymeren oder Copolymeren von Acryl- oder Methacrylsäureestern oder Acryl- oder Methacrylamiden mit aminierten Funktionen, kationischen Polysacchariden, quartären Copolymeren von Vinylpyrrolidon und Vinylimidazol und Chitosanen ausgewählt ist.

**6.** Zusammensetzung nach Anspruch 4, **dadurch gekennzeichnet, dass** das anionische fixierende Polymer unter den Homopolymeren oder Copolymeren von Acrylsäure und Methacrylsäure oder deren Salzen, Copolymeren von

Crotonsäure, Copolymeren von einfach ungesättigten Carbonsäuren oder Carbonsäureanhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und gepfropften anionischen Siliconpolymeren ausgewählt ist.

**7.** Zusammensetzung nach Anspruch 4, **dadurch gekennzeichnet, dass** das amphotere fixierende Polymer unter den Copolymeren mit sauren Vinyleinheiten und basischen Vinyleinheiten, vernetzten und acylierten Polyaminoamiden, Polymeren mit zwitterionischen Einheiten, von Chitosan abgeleiteten Polymeren, modifizierten Alkyl($C_{1-5}$) vinylether/Maleinsäureanhydrid-Copolymeren, amphoteren Polyurethanen und amphoteren gepfropften Siliconpolymeren ausgewählt ist.

**8.** Zusammensetzung nach Anspruch 4, **dadurch gekennzeichnet, dass** das nichtionische fixierende Polymer unter den Polyalkyloxazolinen, Homopolymeren und Copolymeren von Vinylacetat, Homopolymeren und Copolymeren von Acrylsäureestern, Acrylnitril-Copolymeren, Homopolymeren und Copolymeren von Styrol, Polyamiden, Homopolymeren von Vinyllactam, die von Homopolymeren von Vinylpyrrolidon verschieden sind, Copolymeren von Vinyllactam, nichtionischen Polyurethanen und nichtionischen gepfropften Siliconpolymeren ausgewählt ist.

**9.** Kosmetische Zusammensetzung nach einem der Ansprüche 4 bis 8, wobei die Konzentration des oder der ergänzenden fixierenden Polymere im Bereich von 0,1 bis 20 % und vorzugsweise 0,5 bis 10 Gew.-%, bezogen auf das Gesamtgewicht der Zusammensetzung, liegt.

**10.** Kosmetische Zusammensetzung nach einem der Ansprüche 4 bis 8, wobei die in den erfindungsgemäßen Zusammensetzungen verwendeten organischen Lösungsmittel niedere $C_{1-4}$-Alkohole, wie Ethanol, Isopropanol, *tert*-Butanol und *n*-Butanol, oder Polyole oder Polyolether sein können.

**11.** Kosmetische Zusammensetzung nach einem der vorhergehenden Ansprüche, die mindestens eine Verbindung enthält, die unter den Siliconen, siliconierten Fettsubstanzen und nichtsiliconierten Fettsubstanzen ausgewählt ist.

**12.** Kosmetische Zusammensetzung nach Anspruch 11, wobei die Konzentration der Verbindung(en), die unter den Siliconen, siliconierten Fettsubstanzen und nichtsiliconierten Fettsubstanzen ausgewählt ist (sind), im Bereich von 0,01 bis 20 % und vorzugsweise 0,05 bis 10 Gew.-%, bezogen auf das Gesamtgewicht der Zusammensetzung, liegt.

**13.** Kosmetische Zusammensetzung nach einem der vorhergehenden Ansprüche, wobei sie mindestens einen Zusatzstoff enthält, der unter den nichtionischen, anionischen kationischen und amphoteren grenzflächenaktiven Stoffen, ergänzenden nichtionischen, anionischen, kationischen amphoteren Polymeren, Ceramiden und Pseudoceramiden, Vitaminen und Provitaminen, darunter Panthenol, wasserlöslichen und fettlöslichen, siliconierten oder nichtsiliconierten Sonnenschutzfiltern, Füllstoffen und festen Partikeln, wie beispielsweise anorganischen und organischen, farbigen oder nicht farbigen Pigmenten, Perlglanzstoffen und Trübungsmitteln, Pailletten, wirksamen Partikeln, Farbmitteln, Maskierungsmitteln, Weichmachern, Solubilisierungsmitteln, Ansäuerungsmitteln, Alkalisierungsmitteln, Neutralisationsmitteln, anorganischen und organischen Verdickungsmitteln, Antioxidantien, Hydroxysäuren, Penetrationsmitteln, Parfums und Konservierungsmitteln ausgewählt ist.

**14.** Kosmetische Zusammensetzung nach einem der vorhergehenden Ansprüche, **dadurch gekennzeichnet, dass** sie keinen $C_{1-4}$-Alkohol enthält.

**15.** Verfahren für die Formgebung oder Festigung der Frisur, wobei die kosmetische Zusammensetzung nach einem der Ansprüche 1 bis 14 verwendet wird.

**16.** Verwendung einer kosmetischen Zusammensetzung nach einem der Ansprüche 1 bis 14 für die Fixierung und Festigung der Frisur.

**17.** Verwendung der Zusammensetzung nach Anspruch 16, um eine wasserfeste Frisur zu bilden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 1222944
- DE 2330956
- GB 839805 A
- FR 2350384
- FR 2357241
- FR 2198719
- US 4128631 A
- EP 080976 A
- FR 2077143
- FR 2393573
- US 4131576 A
- FR 1400366
- EP 0412704 A
- EP 0412707 A
- EP 0640105 A
- WO 9500578 A
- EP 0582152 A
- WO 9323009 A
- US 4693935 A
- US 4728571 A
- US 4972037 A
- FR 8516334 A
- US 4957732 A
- EP 186507 A
- EP 342834 A
- JP 2295912 A
- FR 2416723
- US 2798053 A
- US 2923692 A
- US 2528378 A
- US 2781354 A
- US 4874554 A
- US 4137180 A


### Littérature non-brevet citée dans la description


- **WALTER NOLL.** Chemistry and Technology of Silicones. Academic Press, 1968
- **TODD ; BYERS.** Volatile Silicone fluids for cosmetics. *Cosmetics and toiletries,* Janvier 1976, vol. 91, 27-32
- **M.R. PORTER.** Handbook of Surfactants. Blackie & Son, 1991, 116-178